# EUROPEAN PATENT SPECIFICATION

(11) **EP 3 173 077 B1**
(45) Date of publication and mention of the grant of the patent: **06.05.2020**
(21) Application number: 16201022.7
(22) Date of filing: 29.11.2016
(51) Int. Cl.: A61K 9/20, A61K 31/18, A61K 31/704

(54) **TABLET FORMULATIONS OF NIMESULIDE AND THIOCOLCHICOSIDE**
TABLETTENFORMULIERUNGEN AUS NIMESULID UND THIOCOLCHICOSID
FORMULATIONS DE COMPRIMÉS DE NIMÉSULIDE ET DE THIOCOLCHICOSIDE

(30) Priority: 30.11.2015 TR 201515144
(43) Date of publication of application: 31.05.2017
(73) Proprietor: Sanovel Ilac Sanayi ve Ticaret A.S., 34460 Istanbul (TR)
(72) Inventor: TÜRKYILMAZ, Ali, 34460 Istanbul (TR); YILDIRIM, Ediz, 34460 Istanbul (TR); DEMIR, Bülent, 34460 Istanbul (TR)
(74) Representative: Sevinç, Erkan

(56) References cited:
- WO-A1-2010/120253
- WO-A1-2014/125085

## Description

### Technical Field

The present invention relates to a monolayer tablet formulation comprising nimesulide, thiocolchicoside, lactose monohydrate and microcrystalline cellulose and one or more pharmaceutically acceptable excipients.

### Background of Invention

Nimesulide is a non-steroidal anti-inflammatory drug (NSAID) molecule with the chemical structure illustrated in Formula 1 below.

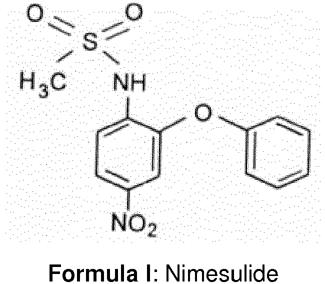

Nimesulide selectively inhibits the cyclooxygenase-2 (COX-2) enzyme. It has antipyretic, analgesic, and anti-inflammatory effects. It is used in osteoarthritis and extraarticular rheumatic diseases, post-traumatic and post-operative inflammations and painful symptoms, high fever, and in dysmenorrheal.

Thiocolchicoside is a myorelaxant with the structure illustrated in Formula 2 below.

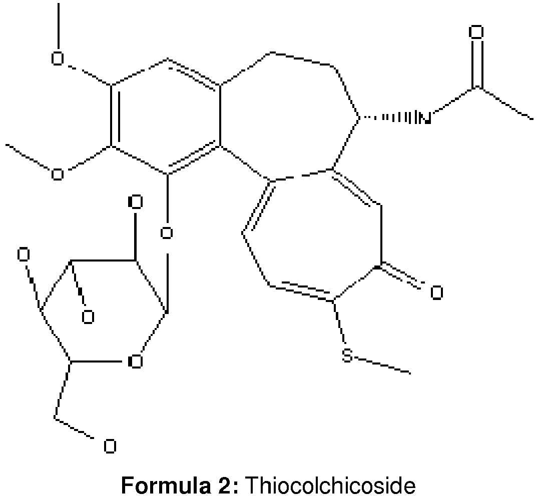

Thiocolchicoside is a semi-synthetic sulfur salt, obtained from colchicoside, a natural glycoside found in Anatolian Colchicum Autumnale (*Autumn crocuses*). This natural glycoside has muscle relaxant, anti-inflammatory and analgesic effects. Thiocolchicoside exerts the myorelaxant effect by activating the gamma-aminobutyric acid (GABA) and glycine receptors at the spinal level.

Furthermore, glycinomimetic effects of thiocolchicoside are seen in the nerve system at varying levels. It does not have a curarizing effect. It does not give rise to motor plaque paralysis, and no inhalation-related problems are encountered. It does not have effects on the cardiovascular system either.

Muscle relaxants also reduce the muscle tonus and are used in treating muscle spasms and contractures. Muscle spasm is one of the main factors held responsible for chronic pains; in addition to rheumatic inflammatory and degenerative orthopedic pathologies, it defines various pathologies of the locomotor system as well; when it affects the joints, it does not cause pain only, but it leads to stiffness that reduces the mobility and flexibility of joints at the affected site.

Muscle contractures also characterizes various pathologies of the locomotor system, and is one of the main causes, which is deemed responsible for the persistency of pain associated with such pathologies.

Muscle relaxants are used in neuromuscular and muscle-skeleton system injuries. There are two main types of muscle relaxants: centrally-acting muscle relaxants and directly-acting muscle relaxants.

Centrally-acting muscle relaxants typically act on the central nervous system (CNS) in a selective manner and are primarily used for alleviating painful muscle spasms and the strains occurring during muscle-skeleton system and neuromuscular damages. The action mechanisms thereof are associated with the causes of the CNS-suppressing activities.

Accordingly, muscle relaxants and antispasmodic molecules constitute a subject matter which is still clinically significant.

Recently, it has been reported that the activity of thiocolchicoside was based on its capability of interacting with strychnine-sensitive glycine receptors, and therefore compounds with glycinomimetic effects have been introduced for use in rheumatologic-orthopedic fields as muscle relaxants.

Muscle relaxants are used alone or together with customary analgesics in treating pain. In fact, a pharmaceutical combination may give complex or unforeseeable outcomes; but so far, nimesulide has never been used together with thiocolchicoside in a monolayer pharmaceutical tablet for treating inflammatory, pain, and muscle-skeletal system diseases.

While combining more than one molecule in one dosage form is increasing the patients' quality of life, many challenges also occur such as the physicochemical compatibility and the therapeutic compatibility between the two active agents. It is essential to achieve the compatibility between different active agents and/or between active agents and excipients used. Compounds of different classes cannot always be combined into safe and efficacious dosage forms thereby resulting in incompatible drug combinations. Due to chemical interaction between active agents or between active agents and excipients, stability and release problems may occur.

Researching the patent literature may result in various patents which relate to nimesulide and thiocolchicoside.

The patent application WO2014125085A1 discloses a multilayer oral tablet formulation comprising nimesulide or a pharmaceutically acceptable salt thereof and thiocolchicoside or a pharmaceutically acceptable salt thereof. The application comprises a stable and well-soluble combination of nimesulide and thiocolchicoside and the layer comprising nimesulide and the layer comprising thiocolchicoside are separated using an inert layer to overcome incompatibility and safety problems.

In prior art, trilayer tablet is used to overcome stability problems of combination of nimesulide and thiocolchicoside. However, conventional monolayer tablets have more advantages than trilayer tablets in terms of easy and economical process and patient compliance. Therefore, there is a need to develop a monolayer tablet formulation comprising nimesulide and thiocolchicoside.

### Detailed Description of Invention

The present invention relates to a monolayer tablet formulation comprising nimesulide, thiocolchicoside, lactose monohydrate and microcrystalline cellulose and one or more pharmaceutically acceptable excipients.

An embodiment of this present invention is to obtain a monolayer tablet formulation comprising nimesulide and thiocolchicoside by an easy process. In prior art, there is a trilayer tablet comprising nimesulide and thiocolchicoside. However, in this present invention monolayer tablet has been chosen to combine nimesulide and thiocolchicoside. It has been found that monolayer tablet comprising nimesulide and thiocolchicoside has easier and more economical process than the trilayer tablet comprising nimesulide and thiocolchicoside. Trilayer tablet has steps for each layer. However, monolayer tablet process of combination of nimesulide and thiocolchicoside has few steps for just one layer and it takes less time.

In this embodiment of this present invention, for patients, monolayer tablet comprising nimesulide and thiocolchicoside has been found more convenient as compared to prior art. Application of monolayer tablet comprising nimesulide and thiocolchicoside is easier for patients who have difficulties swallowing than multilayer tablet due to its weight and volume. It has been found that during the process of monolayer tablet of nimesulide and thiocolchicoside combination, less excipient has been used. Using less excipients gives monolayer tablet advantages such as having less weight and volume and having less side effects caused by excipients.

In this embodiment, it is difficult to solubilize active agents by using less excipients. To achieve solubility of both nimesulide and thiocolchicoside, specific particle size of lactose monohydrate has been used. In that way, it has been surprisingly found that solubility problem of nimesulide and thiocolchicoside has been overcome.

According to this embodiment, particle size of lactose monohydrate in diameter range with a d50 value is between 10 to 200 µm, preferably 30 to 150 µm and more preferably it is 60 to 105 µm.

According to this embodiment, particle size of lactose monohydrate in diameter range with a d90 value is between 10 to 200 µm, preferably 20 to 100 µm and more preferably it is 20 to 40 µm.

In this embodiment of this present invention, the disintegrant is microcrystalline cellulose (PH101).

According to this embodiment, while using less excipient to develop monolayer tablet formulation comprising nimesulide and thiocolchicoside, it is difficult to provide desired dissolution. To achieve desired dissolution profile of nimesulide and thiocolchicoside, specific particle size of microcrystalline cellulose has been used to increase solubility of both active agent. In that way, it has been surprisingly found that desired dissolution of nimesulide and thiocolchicoside has been achieved, while stability of the formulation remained intact.

According to this embodiment, particle size of microcrystalline cellulose in diameter range with a d50 value is between 10 to 200 µm, preferably 20 to 100 µm and more preferably it is 40 to 80 µm.

According to this embodiment, particle size of microcrystalline cellulose in diameter range with a d90 value is between 10 to 200 µm, preferably 30 to 200 µm and more preferably it is 70 to 160 µm.

According to one embodiment, the monolayer tablet formulation comprising;
a) 10.0 - 30.0% Nimesulid,
b) 0.1 - 5.0% Thiocolchicoside,
c) 1.0 - 50.0% Lactose monohydrate,
d) 1.0 - 30.0% Microcrystalline cellulose,
e) 1.0 - 15.0% Sodium starch glycolate,
f) 0.01 - 5.0% Hydroxypropyl cellulose,
g) 0.01 - 3.0% Magnesium stearate by weight of total composition.

According to this embodiment, the monolayer tablet formulation comprising;
a) 25.0 % Nimesulid,
b) 2.0 % Thiocolchicoside,
c) 37.8 % Lactose monohydrate,
d) 25.0 % Microcrystalline cellulose,
e) 8.75 % Sodium starch glycolate,
f) 0.20 % Hydroxypropyl cellulose,
g) 1.25 % Magnesium stearate by weight of total composition.

In a preferred embodiment, the monolayer tablet formulation comprising;
a) Nimesulide,
b) Thiocolchicoside,
c) Lactose monohydrate in diameter range with a d50 value is between 10 to 200 µm, preferably 30 to 150 µm and more preferably it is 60 to 105 µm,
d) Microcrystalline cellulose in diameter range with a d50 value is between 10 to 200 µm, preferably 20 to 100 µm and more preferably it is 40 to 80 µm.

In another preferred embodiment, the monolayer tablet formulation comprising;
a) Nimesulide or a pharmaceutically acceptable salt,
b) Thiocolchicoside or a pharmaceutically acceptable salt,
c) Lactose monohydrate in diameter range with a d90 value is between 10 to 200 µm, preferably 20 to 100 µm and more preferably it is 20 to 40 µm,
d) Microcrystalline cellulose in diameter range with a d90 value is between 10 to 200 µm, preferably 30 to 200 µm and more preferably it is 70 to 160 µm.

In this present invention, particle sizes of lactose monohydrate and microcrystalline cellulose have been measured by *Malvern Mastersizer 2000 laser diffraction particle size analyzer* with solid samples and d50 value of these excipients have been identified. "d50" or "d(0.5)" is defined as the median diameter or the medium value of the particle size distribution, it is the value of the particle diameter at 50% in the cumulative distribution. "d90" or "d(0.9)" is defined as the size value corresponding to volume of the particles at 90%, which represents the size of particles below which 90% of the substance lies.

Suitable binders for use in the formulation according to the present invention are natural gums, starch, gelatin, polyvinylpyrrolidone, polymethacrylates; collagen, proteins such as gelatin; semisynthetic polymers such as agar, alginate, sodium alginate, pectin, starch, carboxymethyl cellulose, hydroxypropyl cellulose, methyl cellulose; synthetic polymers such as carbomer, poloxamer, polyacrylamide, polyvinyl alcohol; inorganic substances such as aluminum hydroxide, bentonite, laponite; starch mucilage, acacia mucilage, polydextrose, polyethylene oxide and mixtures thereof.

Suitable lubricants/glidants for use in the formulation according to the present invention are sodium stearyl fumarate, magnesium stearate, polyethylene glycol, stearic acid, metal stearates, boric acid, sodium chloride benzoate and acetate, sodium or magnesium lauryl sulfate and mixtures thereof.

Suitable sweeteners for use in the formulation according to the present invention are sucralose, acesulfame-K, aspartame, saccharine or saccharine sodium and calcium salts, sodium cyclamate, sucrose, fructose, glucose, sorbitol and mixtures thereof.

Suitable flavoring agents for use in the formulation according to the present invention are fruit aromas such as orange, banana, strawberry, cherry, wild cherry, lemon; cardamom, anis, mint, menthol, vanillin, and ethyl vanillin, and other similar aromas and mixtures thereof.

The present invention is used for treating osteoarthritis, pain associated with tissue trauma following osteoarthritis surgery, psoriatic arthritis, rheumatoid arthritis, myalgia, bone pain, arthralgia, muscle spasms, soft tissue traumas, lumbago, back pain, sciatica and torticollis.

### Example

| | **amount (mg)** | **amount (%)** |
|---|---|---|
| Nimesulid | 100.0 mg | % 25.00 |
| Thiocolchicoside | 8.0 mg | % 2.00 |
| Lactose monohydrate | 151.2 mg | %37.80 |
| Microcrystalline cellulose | 100.0 mg | % 25.00 |
| Sodium starch glycolate | 35.0 mg | % 8.75 |
| Hydroxypropyl cellulose | 0.8 mg | % 0.20 |
| Magnesium stearate | 5.0 mg | % 1.25 |
| **Total weight** | **400.00 mg** | |

The production of the formulation is carried out as follows:
Nimesule, thiocolchicoside, lactose monohydrate, microcrystalline cellulose and sodium starch glycolate are weighed and loaded into Aeromatic. Hydroxypropyl cellulose is dissolved in water. With this solution granulation is performed. Granules are dried and sieved. Magnesium stearate is added and tablet compression is performed.

## Claims

1. A monolayer tablet formulation comprising nimesulide, thiocolchicoside, lactose monohydrate and microcrystalline cellulose and one or more pharmaceutically acceptable excipients.

2. The monolayer tablet formulation according to claim 1, wherein particle size of lactose monohydrate in diameter range with a d50 value is between 10 to 200 µm, preferably 30 to 150 µm and more preferably it is 60 to 105 µm.

3. The monolayer tablet formulation according to claim 1, wherein particle size of lactose monohydrate in diameter range with a d90 value is between 10 to 200 µm, preferably 20 to 100 µm and more preferably it is 20 to 40 µm.

4. The monolayer tablet formulation according to claim 1, wherein particle size of microcrystalline cellulose in diameter range with a d50 value is between 10 to 200 µm, preferably 20 to 100 µm and more preferably it is 40 to 80 µm.

5. The monolayer tablet formulation according to claim 1, wherein particle size of microcrystalline cellulose in diameter range with a d90 value is between 10 to 200 µm, preferably 30 to 200 µm and more preferably it is 70 to 160 µm.

6. The monolayer tablet formulation according to any preceding claims, comprising;
a) 10.0 - 30.0% Nimesulide,
b) 0.1 - 5.0% Thiocolchicoside,
c) 1.0 - 50.0% Lactose monohydrate,
d) 1.0 - 30.0% Microcrystalline cellulose,
e) 1.0 - 15.0% Sodium starch glycolate,
f) 0.01 - 5.0% Hydroxypropyl cellulose,
g) 0.01 - 3.0% Magnesium stearate by weight of total composition.

## Patentansprüche

1. Eine Monolayer-Tablettenformulierung umfassend Nimesulid, Thiocolchicosid, Lactose-Monohydrat und mikrokristalline Cellulose sowie einen oder mehrere pharmazeutisch verträgliche Hilfsstoffe.

2. Monolayer-Tablettenformulierung nach Anspruch 1, wobei die Teilchengröße von Lactosemonohydrat im Durchmesserbereich mit einem d50-Wert zwischen 10 und 200 µm, vorzugsweise 30 bis 150 µm und noch bevorzugter 60 bis 105 µm beträgt.

3. Monolayer-Tablettenformulierung nach Anspruch 1, wobei die Teilchengröße von Lactosemonohydrat im Durchmesserbereich mit einem d90-Wert zwischen 10 und 200 µm, vorzugsweise 20 bis 100 µm und noch bevorzugter 20 bis 40 µm beträgt.

4. Monolayer-Tablettenformulierung nach Anspruch 1, wobei die Teilchengröße von mikrokristalliner Cellulose im Durchmesserbereich mit einem d50-Wert zwischen 10 und 200 µm, vorzugsweise 20 bis 100 µm und noch bevorzugter 40 bis 80 µm beträgt.

5. Monolayer-Tablettenformulierung nach Anspruch 1, wobei die Teilchengröße von mikrokristalliner Cellulose im Durchmesserbereich mit einem d90-Wert zwischen 10 und 200 µm, vorzugsweise 30 bis 200 µm und noch bevorzugter 70 bis 160 µm beträgt.

6. Die Monolayer-Tablettenformulierung nach allen vorhergehenden Ansprüchen, umfassend;
a) 10,0- 30,0% Nimesulid,
b) 0,1 -5,0% Thiocolchicosid,
c) 1,0 - 50,0% Lactosemonohydrat,
d) 1,0 - 30,0% mikrokristalline Zellulose,
e) 1,0- 15,0% Natriumstärkeglykolat,
f) 0,01 -5,0% Hydroxypropyl-Zellulose,
g) 0,01 -3,0% Magnesiumstearat nach Gewicht der Gesamtzusammensetzung

## Revendications

1. Formulation de comprimé monocouche comprenant du nimésulide, du thiocolchicoside, du lactose monohydraté et de la cellulose microcristalline et un ou plusieurs excipients pharmaceutiquement acceptables.

2. Formulation de comprimé monocouche selon la revendication 1, dans laquelle la taille des particules de lactose monohydraté dans la plage de diamètre avec une valeur d50 est entre 10 et 200 µm, de préférence entre 30 et 150 µm et de façon davantage préférée elle est de 60 à 105 µm.

3. Formulation de comprimé monocouche selon la revendication 1, dans laquelle la taille des particules de lactose monohydraté dans la plage de diamètre avec une valeur d90 est entre 10 et 200 µm, de préférence entre 20 et 100 µm et de façon davantage préférée elle est de 20 à 40 µm.

4. Formulation de comprimé monocouche selon la revendication 1, dans laquelle la taille des particules de cellulose microcristalline dans la plage de diamètre avec une valeur d50 est entre 10 et 200 µm, de préférence entre 20 et 100 µm et de façon davantage préférée elle est de 40 à 80 µm.

5. Formulation de comprimé monocouche selon la revendication 1, dans laquelle la taille des particules de cellulose microcristalline dans la plage de diamètre avec une valeur d90 est comprise entre 10 et 200 µm, de préférence entre 30 et 200 µm et de façon davantage préférée elle est de 70 à 160 µm.

6. Formulation de comprimé monocouche selon l'une quelconque des revendications précédentes, comprenant :
a) 10,0 - 30,0 % de Nimésulide ;
b) 0,1 - 5,0 % de Thiocolchicoside ;
c) 1,0 - 50,0% de Lactose monohydraté ;
d) 1,0 - 30,0% de Cellulose microcristalline ;
e) 1,0 - 15,0 % de Glycolate d'amidon sodique ;
f) 0,01 - 5,0 % d'Hydroxypropyl cellulose ;
g) 0,01 - 3,0 % de Stéarate de magnésium en poids de la composition totale.
